# EUROPEAN PATENT APPLICATION

(11) **EP 4 826 735 A1**
(43) Date of publication of application: **21.10.2026**
(21) Application number: 26167740.5
(22) Date of filing: 25.03.2026
(51) Int. Cl.: G16H 40/63, G16H 40/67, G16H 50/20, G16H 50/30, G16H 50/70

(54) **CONDITIONAL ALARMS FOR MONITORING PATIENT DATA**

(30) Priority: 17.04.2025 US 202519182377
(71) Applicant: GE Precision Healthcare LLC, Waukesha, WI 53188 (US)
(72) Inventor: KESAVALU, Baskar, 560037 Bengaluru (IN); AMMANNA, Shivakumar, 560066 Bengaluru (IN); PRADYOTH, Pavana, 560066 Bengaluru (IN); BHASKAR, Madhusudan, 560066 Bengaluru (IN); PEDRIN, Sushma Sharon, Bengaluru, 560066 (US); CHRISTOPHER SELVI, Godwin, Bengaluru, 560066 (US)
(74) Representative: Kilburn & Strode LLP

(57) **Abstract**

Systems and methods are proposed to address the issue of alarm fatigue among caregivers attending to patients who are being monitored using patient monitors that are configured to generate alarms when measured physiological parameters exceed threshold values. A centralized multi-condition alarm system (260) is disclosed that allows caregivers to selectively silence some alarms configured for some patient monitors (110), while allowing other alarms configured for other patient monitors (110) to be generated when conditions are met. This feature would allow some alarms to be generated when their parameters and/or configurable combinations of parameters exceed defined thresholds, while silencing other alarms when their parameters and/or configurable combinations of parameters exceed defined thresholds. This may reduce non-actionable alarms and enhance early detection of patient deterioration.

## Description

### TECHNICAL FIELD

Embodiments of the subject matter disclosed herein relate to patient monitoring, and in particular to configuring alarms generated by patient monitors.

### BACKGROUND

Patients monitored in critical care units (CCU) may be kept under surveillance with continuous monitoring of their physiological parameters. Clinicians may be notified when deviations in a physiological parameter are detected by an alarm, which may be automatically triggered in response to the physiological parameter exceeding a threshold value. However, due to a large number of patients being treated within a unit and a large number of parameters that may be monitored, a number of alarms that is generated may be high. In such cases, alarm fatigue due to repeated triggering of alarms for non-critical medical events may affect responsiveness of medical professionals. Additionally, having repeated alarms that are not attended to wastes memory and processing resources of patient monitoring systems.

### SUMMARY

In one example, the problems described above may be addressed by a multi-condition alarm system for a patient monitoring system, comprising a processor and a memory storing instructions that when executed, cause the processor to receive physiological parameters of a plurality of patients via a respective plurality of patient monitors coupled to the plurality of patients; generate a plurality of multi-condition alarms for the plurality of patients, each multi-condition alarm configured to output a respective audio signal when two or more physiological parameters of a respective patient exceed predefined thresholds concurrently; in response to an input from a user of the multi-condition alarm system, configure a first portion of the plurality of multi-condition alarms to be silenced when two or more physiological parameters of a respective patient exceed predefined thresholds concurrently, and configure a second portion of the plurality of multi-condition alarms not to be silenced when two or more physiological parameters of a respective patient exceed predefined thresholds concurrently; in a first condition where two or more physiological parameters of a first multi-condition alarm of the first portion of multi-condition alarms exceed respective predefined thresholds concurrently, not output the respective audio signal; and in a second condition where two or more physiological parameters of a second multi-condition alarm of the second portion of multi-condition alarms exceed respective predefined thresholds concurrently, output the respective audio signal.

The above advantages, other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a pictorial view of an exemplary patient monitoring system;
FIG. 2 is a schematic diagram of the patient monitoring system;
FIG. 3 is an exemplary work flow diagram for a patient monitoring process;
FIG. 4 is a schematic diagram illustrating exemplary alarms that may be generated during patient monitoring;
FIG. 5 is a flowchart illustrating an exemplary high-level method for configuring how various alarms may be generated during patient monitoring via a patient monitor;
FIG. 6 is a flowchart illustrating an exemplary method for configuring an alarm based on parameter thresholds;
FIG. 7 is a flowchart illustrating an exemplary method for updating an active alarm, including silencing the active alarm;
FIG. 8 is a flowchart illustrating an exemplary method for generating an alarm based on patient physiological data;
FIG. 9 shows an exemplary user interface of a patient monitoring system;
FIG. 10 shows an exemplary user interface of a multi-condition alarm system; and
FIG. 11 is a schematic diagram showing an exemplary flow of data when a template recommendation system is used to generate a template of a multi-condition alarm.

### DETAILED DESCRIPTION

This description and embodiments of the subject matter disclosed herein relate to monitoring physiological patient data, meaning, time-series data acquired over regular time intervals via electrophysiological monitoring. The phrase "time-series data" can refer to a sequence of data that is repeatedly generated and/or captured by a device (e.g., a computing device) at a plurality of time values during a certain time interval. Examples of time-series data include the continuous monitoring of a person's heart rate, blood pressure, etc.

Electrophysiological monitoring is a common way to assess the health of a patient and to diagnose and/or detect the presence of an adverse health condition based on physiological time-series data. Sometimes, the electrophysiological monitoring detects anomalies or abnormalities in the time-series data. In some cases, the anomalies may trigger alerts and/or alarms in software programs that monitor the data. Thresholds may be established for different physiological parameters of the patient (also referred to herein as parameters), and the alerts and/or alarms may be generated when a parameter exceeds a respective threshold. For example, an alarm generation system may generate a first alarm if a first physiological parameter of a patient exceeds a first threshold; the alert generation system may generate a second alarm if a second physiological parameter of the patient exceeds a second threshold; the alert generation system may generate a third alarm if a third physiological parameter of the patient exceeds a third threshold; and so on. Such alarms have the potential to alert caregivers to early signs of patient deterioration by highlighting situations where parameters deviate from normal ranges. This could lead to timely interventions and improved patient outcomes.

For the purposes of this disclosure, the term "alarm" refers to an audio output of a speaker, such as a tone, sound, a recorded verbal message, or a different type of audio output. The term "alert" refers to a visual indication that may be displayed, for example, at a bedside device, patient monitor worn by the patient, computer monitor of a care unit or nurse's station, etc. An alert may be accompanied by an alarm, meaning, when a parameter of a patient exceeds a threshold, an alert and an alarm may both be generated concurrently to communicate a potential health concern of the patient to caregivers monitoring the patient.

An alarm is typically configured and generated at a patient monitor arranged on a body of a patient, at a bedside device connected to a patient monitor, or to sensors arranged on the patient body. For example, a first alarm may be configured at a first patient monitor of a first patient, to be generated at the first patient monitor; a second alarm may be configured at a second patient monitor of a second patient, to be generated at the second patient monitor; and so on. In some examples, a plurality of patient monitors may additionally be communicatively coupled to one or more centralized monitoring systems, such as a computer system at a nurse's station of a care unit, and/or edge devices, such as a remote computing device or phone of a caregiver. When an alarm is generated at a patient monitor, a corresponding alarm (e.g., the same alarm) may additionally be generated at the one or more centralized monitoring systems and/or remote computing devices. As a result, caregivers may monitor a plurality of patients from a central location or a different location than a specific patient.

However, the caregivers may have limited or no control over the alarms at the patient monitors from the central location. That is, the caregivers may not be able to turn off an alarm at a patient monitor from a remote location, and the alarms at the patient monitors may have to be switched off manually at the patient. This ensures that a caregiver physically attend to the patient when an alarm is triggered. In some cases, the central monitoring systems may be configured to allow a caregiver to turn off a plurality of alarms at once for a predefined duration (e.g., two minutes). However, such practices incur risks and may be discouraged.

Because alarms may be configured to be generated for a plurality of parameters, various alarms may be generated for a single patient. The various alarms may be generated concurrently, in an overlapping fashion, and/or at different times. Alarms may also be generated for various patients in a same care unit. As a result, clinicians working in the care unit may be exposed to alarms on a regular or continuous basis. This may generate alarm fatigue, where a clinician may become accustomed to hearing alarms and as a result, an attentiveness and/or responsiveness of the caregiver to the alarms may be diminished. Alarm fatigue may result in a health concern of a patient going unnoticed or unattended, which may lead to an undesirable outcome.

In some cases, the alarm fatigue may cause caregivers to turn off alarms generated at one or more patient monitors. However, turning off alarms increases a possibility that a health concern of a patient may not be attended to by a caregiver. Additionally, caregiver workflows are negatively impacted by constant turning of alarms on and/or off, and a cognitive load of the caregivers is increased by having to remember when alarms are turned off.

The proposed system addresses the issue of alarm fatigue among caregivers, with a centralized multi-condition alarm system that allows caregivers to create multi-condition alarm configurations. The multi-condition alarm configurations allow alarms to be generated when specific combinations of parameters exceed respective thresholds concurrently, as opposed to when a single parameter exceeds a threshold value. For example, an alarm may be configured to be generated if both a heart rate (HR) of a patient exceeds a first threshold and a blood pressure (BP) exceeds a second threshold, but not be generated if one of the HR and the BP exceeds a respective threshold and not the other. By offering the multi-condition alarm configurations, a number of individual alarms, and a total number of alarms in a care unit may be reduced. In other words, since many patient parameters have trends that are related, the proposed system may allow caregivers to configure a multi-condition alarm to be generated instead of two or more separate individual-parameter alarms, meaning that the two or more separate individual-parameter alarms may be replaced by the multi-condition alarm.

The multi-condition alarms would be different alarms from individual-parameter alarms set at the patient monitors, and the multi-condition alarms could be set via a remote multi-condition alarm system accessible via various computing devices or computer terminals. An additional advantage of the multi-condition alarms is that because they can be configured via a single user interface (UI) of the remote multi-condition alarm system, a selective silencing feature may be additionally provided, where some multi-condition alarms based on patient parameter data received from a first set of respective patient monitors may be selectively silenced, while other multi-condition alarms based on patient parameter data received from a second set of respective patient monitors may be generated when conditions are met. This is in contrast with the individual alarms, which may not be able to be silenced remotely. Thus, the proposed multi-condition alarm system allows for some of the individual alarms to be replaced by the multi-condition alarms, which may reduce non-actionable alarms and enhance early detection of patient deterioration. The system may also utilize predefined templates for multi-condition alarms, allowing clinicians to configure alarm settings efficiently and consistently across different patient scenarios.

The proposed multi-condition alarm system is communicatively coupled to a plurality of patient monitors, where patient parameter data acquired by the plurality of monitors is transmitted to the multi-condition alarm system. A caregiver may view active multi-condition alarms that have been configured for one or more patient monitors via the UI of the multi-condition alarm system at a centralized location. The caregiver may select to silence a first portion of the active alarms corresponding to a first subset of the plurality of patient monitors, without affecting a second portion of the active alarms corresponding to a second subset of the plurality of patient monitors that will continue to monitor patients and generate alarms as configured, or the individual-parameter alarms typically set at patient bedside. In this way, a centralized control over alarms may be exercised that does not interfere with standard operating procedure.

Further, when an alarm is silenced, the proposed multi-condition alarm system may allow a user to define one or more breakthrough conditions that allow a multi-condition alarm to be generated even when the multi-condition alarm is silenced. For example, at a first time, a caregiver may configure a multi-condition alarm to be generated when a combination of parameters of a patient exceed threshold values in combination. At a second, later time, the caregiver (or a different caregiver attending to the patient) may decide that the combination of parameters is no longer a priority with respect to the patient's health condition, and may decide to silence the alarm. When the caregiver silences the alarm, the caregiver may define one or more breakthrough alarms that are generated if one or more parameters of the combination of parameters exceed breakthrough threshold values. The breakthrough threshold values may be set outside a normal range of threshold values, for example. In this way, an additional "safety net" is provided to ensure that silenced alarms do not hide concerning trend in a parameter or unexpected changes in a patient's condition.

Additionally, when alarms persistently sound without differentiation, caregivers may become desensitized, potentially missing alarms or delaying response. To address this, the multi-condition alarm system may be configured to generate multi-condition alarms of different types, to allow for differentiation between different configured multi-condition alarms. The multi-condition alarms may have different tones, which may be generated based on a priority of a multi-condition alarm, in some examples. For example, a first tone may be generated for a higher priority alarm; a second tone may be generated for a medium priority alarm; and a third tone may be generated for a lower priority alarm, where the first, second, and third tones are different. In addition, a distinctive tone or a short recorded voice message may be generated when certain defined conditions are met for the multi-condition alarm, which may differentiate the multi-condition alarm from other multi-condition alarms.

As a further advantage over existing alarm generation systems, the proposed multi-condition alarm system may allow users to configure alarms based on a rate of change of a parameter. For example, a first conventional alarm may be configured to be generated when a heart rate (HR) of a patient increases above a first threshold (e.g., 100 BPM) or decreases below a second threshold (e.g., 60 BPM). The proposed multi-condition alarm system allows a multi-condition alarm to be generated when a rate of change of the HR increases above a threshold rate of change. For example, if the HR increases from 65 BPM to 80 BPM within a threshold duration, an alarm may be generated, even when the first conventional alarm is not triggered. In this way, alarming trends that are missed by conventional alarm systems may be detected.

Additionally, templates for the multi-condition alarms may be generated and stored, for example, in a centralized template library on a server of a health care system. Caregivers may select and download a desired template for a multi-condition alarm configuration from the centralized template library. The caregivers may edit, adjust and/or customize a template for use with a specific patient. The customized configurations may be added to the centralized template library as new templates, in some examples. By storing the templates in the centralized template library, a higher level of security may be maintained. In some cases, some templates may be additionally stored at an edge device, such as a bedside device (e.g., bedside device 108), and updated from the centralized template library with supervisor approval.

Further, one or more artificial intelligence (AI) systems may be used to recommend templates to caregivers, based on set of conditions of a patient, patient history, caregiver history, or different criteria. In some examples, a third-party AI system (e.g., of a different health care organization, for example) may be used with the proposed alarm generation system. The third-party AI system may rely on an application programmer interface (API) of the alarm generation system, for example). In still other examples, caregivers may interact with the proposed alarm generation system via a natural language processing (NLP) interface, which may allow the caregivers to request a template using natural language. For example, the caregivers may type in a request for a template, or may verbally request a template via a microphone.

In this way, caregivers may more precisely define the types of health concerns that merit alarms, and a total number of alarms that caregivers respond to may be reduced, thereby mitigating alarm fatigue and reducing a consumption of computing resources spent on the alarms. The proposed multi-condition alarm system emphasizes the involvement of clinicians in both prescribing and approving the multi-condition alarm configurations. This ensures that the setup aligns with clinical protocols and prevents errors in configuring alarms that could compromise patient safety. Additionally, the ability to configure alarms based on specific clinical scenarios may allow for customization to suit different patient conditions and monitoring needs.

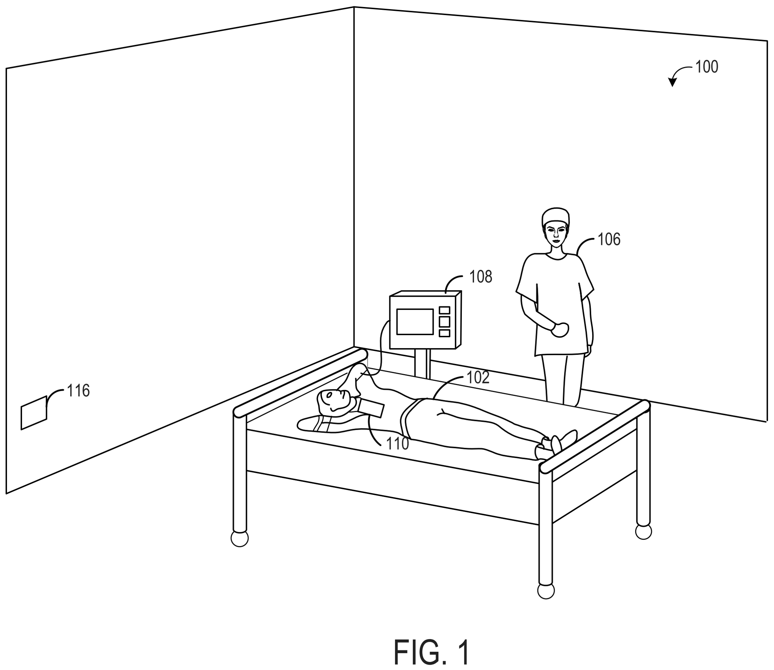

FIG. 1 shows an example patient monitoring environment 100, which in the depicted example is a patient room in a hospital or other medical facility. The patient monitoring environment 100 may include one or more patient monitoring devices, monitoring one or more physiological parameters. The monitoring environment 100 includes a patient 102 being monitored by a plurality of monitoring devices and also being attended to by a clinician 106. Clinician 106 may be a nurse, physician, medical technologist, or another suitable medical professional. The monitoring devices include a bedside device 108 and a patient monitor 110. The monitoring environment 100 depicts the use of more than one monitoring device but it is understood this is non-limiting as the environment 100 could include one device monitoring one parameter, one device monitoring more than one parameter, multiple devices each monitoring one or more than one parameter, and so on. Due to differing patient conditions and the varying patient monitoring needs, one or more devices may be used to support the monitoring needs of the patient and capable of supporting monitoring in various conditions (e.g., in room, in transport, patient ambulation). The one or more bedside devices 108 may be included or mounted in a floor, table top, or roll stand module that includes one or more leads or other components coupled to the patient or in wireless communication to a device connected to the patient, in order to monitor one or more parameters of the patient (such as echocardiograph (ECG) data, respiration, blood pressure, carbon dioxide levels, etc.). The one or more bedside devices 108 are configured to remain in the patient room, and thus patient 102 may have limited mobility when coupled to the one or more bedside devices 108.

Patient monitor 110 may include one or more telemetry devices (with different sensor capabilities) housed in a common module (as shown) or housed in two or more separate modules. Patient monitor 110 may be positioned on the patient (e.g., via a pouch, holder, or similar, attached to a belt of the patient) or on a movable module (e.g., a wheeled module), such that patient monitor 110 may leave the patient room if the patient leaves the patient room, and may travel with the patient. Patient monitor 110 may be connected to the patient 102 via one or more leads or other components or in wireless communication with an associated sensor, in order to monitor one or more parameters of the patient (such as ECG, respiration, blood oxygen level, etc.).

The patient monitoring data collected by patient monitor 110 may be sent wirelessly (e.g., WiFi, Bluetooth, MBAN) and/or via a hard-wired networked connection to one or more associated devices for processing, analysis, storage, display, etc., such as a central station, patient monitoring database, and/or different patient monitoring system. The methods of wireless communication used by patient monitor 110 and the receiving systems vary widely based on the technology used. In one example, communication approach, to facilitate the transfer of the patient monitoring data collected by patient monitor 110, patient monitoring environment 100 and nearby areas (e.g., hallways, closets, open spaces) may include one or more access points 116. Access point 116 may receive and send information (e.g., wirelessly) to patient monitor 110 (e.g., the patient monitoring data, communication status). The access point 116 sends the received information to a processing server, a central station, a telemetry monitoring system, and/or another suitable device. If patient 102 leaves the patient room and moves throughout the medical facility, patient monitoring data collected by patient monitor 110 may be sent to other access points located throughout the medical facility. Patient monitoring data collected by the one or more bedside devices 108 may likewise be sent to a processing and analysis server, the central station, the telemetry monitoring system, and/or another suitable device, via wireless communication with access point 116 or another access point, or via a wired connection. It is understood, this example using a transceiver and access point for data communications is one of many different technologies suitable for sharing acquired patient monitoring data with the associated data processing, analysis, storage, and information viewing system components and infrastructure.

Patient monitor 110 and/or bedside device 108 may be configured to generate an alarm under certain conditions. For example, if one or more physiological parameters of patient 102 being monitored by patient monitor 110 exceed a predefined or caregiver-configured threshold, the alarm may be generated at patient monitor 110 and/or bedside device 108. In some examples, the alarm may alternatively or additionally be generated at a remote device, such as a nurses' station, care unit computer, or personal computing device of a caregiver. The alarm may be configured on patient monitor 110, bedside device 108, or the remote device.

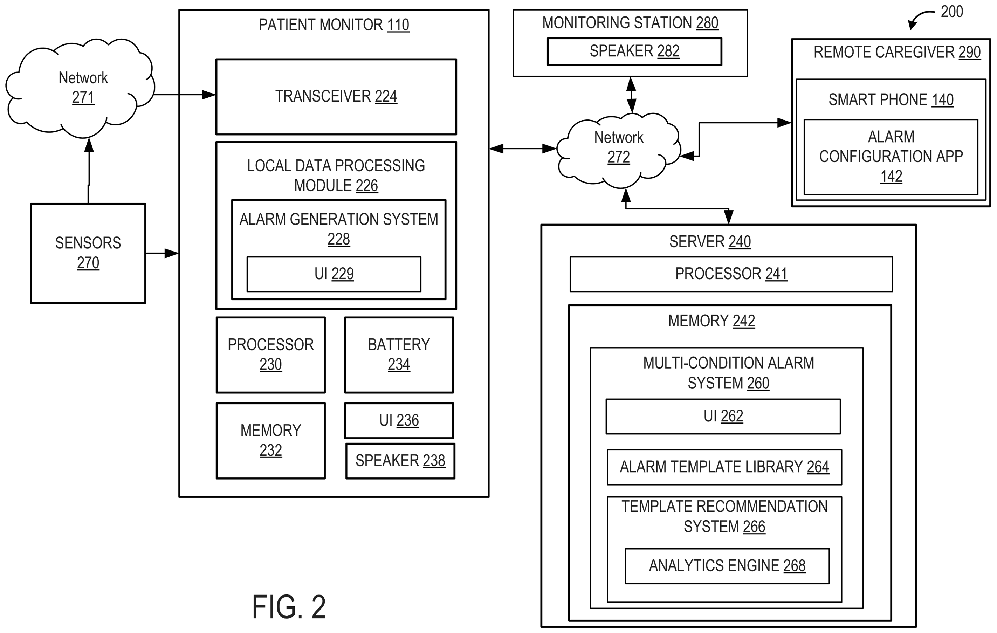

Referring now to FIG. 2, a patient monitoring system 200 is shown including patient monitor 110 of FIG. 1. Patient monitor 110 may be connected to one or more sensors 270, where patient data may be transmitted from the one or more sensors 270 to patient monitor 110 over a network 271 or via a wired connection. Patient monitor 110 may send data to a server 240, and receive data transmitted from server 240 over a network 272, which may be different from network 271. In various embodiments, the patient monitoring system 200 may be established within a hospital environment or healthcare facility, such as within a patient monitoring environment such as the patient monitoring environment 100 of FIG. 1.

The one or more sensors 270 may be specially designed devices for sensing a certain type or types of patient data via placement on a patient's body, and communicating the patient data to patient monitor 110. The one or more sensors 270 may further include a plurality of sensors of different types or the same type. The one or more sensors 270 may include sensors for obtaining physiological patient data from a patient. For example, the one or more sensors may include, but are not limited to, a 3-lead ECG, a pulse oximetry sensor, a blood pressure sensor, a digital stethoscope, a respiratory sensor, a temperature sensor, and the like. The one or more sensors 270 may include a combination of one or more different kinds of sensor.

The physiological patient data is alternatively referred to herein as patient data. The patient data may include, for example, vital signs of the patient, such as a blood pressure or a pulse, and/or any other type of data that may be acquired from a sensor capable of acquiring patient physiological patient data in real-time. Thus, the data sensed by the one or more sensors 270 correlates to the type of sensors in one or more sensors 270 and may include ECG data, blood pressure data, blood oxygen (SPO2) data, respiratory rate, otoscope data, temperature data, and the like. It will be appreciated that the types of sensors listed above are mentioned for illustrative purposes, and the one or more sensors 270 may additionally include other types of sensors for obtaining physiological patient data of a patient without departing from the scope of this disclosure.

Networks 271 and/or 272 may include in a non-limiting manner, a wide area network (WAN); a local area network (LAN); the Internet; a wired or wireless (e.g. optical, Bluetooth, Bluetooth Low Energy (BLE), radio frequency (RF)) network; a cloud-based computer infrastructure of computers, routers, servers, gateways, etc., or any combination thereof associated therewith that allows one or more computing devices within the patient monitoring system 200 to connect with each other. The networks 271 and 272 may include a public network, or a private network associated with a portion of a care facility, for example, a surgery module or department of a hospital, or may be more broadly located across medical devices of an entire hospital or hospital system. In some embodiments, patient monitor 110 and the one or more sensors 270 may be communicatively coupled via a wireless personal area network (PAN) technology such as MBAN. In other embodiments, any PAN technology may be used, such as induction wireless, infrared wireless, ultra wideband (UWB), Bluetooth^{®}, or any other similar technology for wireless communication between co-located devices. For example, the one or more sensors 270 may communicate with patient monitor 110 via an MBAN network of network 271, and patient monitor 110 may communicate with other elements of patient monitoring system 200 via a WiFi network of network 272.

Patient monitor 110 may include a transceiver 224, a local data processing module 226, a processor 230, a memory 232, a battery 234, a user interface (UI) 236, and a speaker 238. Patient monitor 110 may be adapted to receive data over the network 272 via the transceiver 224. The transceiver 224 may also be configured to transmit data over the network 272. In some embodiments, the transceiver 224 may be or may include a WLAN wireless card. In some embodiments, the WLAN card may be an original equipment manufacturer (OEM) card, and may include a storage medium having computer executable code and a processor to execute that code, thus effectuating the operation of the WLAN card. Patient monitor 110 may use the transceiver 224 to connect to the network 272. For example, the transceiver 224 may connect to the network via an access point of the network (e.g., access point 116 of FIG. 1) arranged at a location of the hospital environment, for example, in proximity to patients being monitored at a care module. The network 272 may receive wirelessly transmitted information from the access point, and relay the information to one or more connected devices and/or a hospital information system suitable for collecting and managing such information.

Patient monitor 110 may include a processor 230. The processor 230 may control the operation of patient monitor 110 in response to instructions stored in a memory 232 and/or control signals from the UI 236. In some embodiments, the UI 236 may be integrated into patient monitor 110, where a user may interact with, adjust, or select control elements in the UI 236 (e.g., buttons, knobs, touchscreen elements, etc.) to send one or more control signals to the processor 230 from the UI 236. In other embodiments, the UI 236 is not integrated into patient monitor 110, and the user may interact with, adjust, or select control elements in UI 236 via a user input device, such as a mouse, track ball, touchpad, etc., or the operator may interact with UI 236 via a separate touchscreen, where the operator touches a display screen of UI 236 to interact with UI 236, or via another type of input device. The UI 236 may include a display (e.g., screen or monitor) and/or other subsystems.

Processor 230 may execute instructions stored on memory 232 to control patient monitor 110. As discussed herein, the memory 232 may include any non-transitory computer readable medium in which programming instructions are stored. For the purposes of this disclosure, the term "tangible computer readable medium" is expressly defined to include any type of computer readable storage. The example methods and systems may be implemented using coded instruction (e.g., computer readable instructions) stored on a non-transitory computer readable medium such as a flash memory, a read-only memory (ROM), a random-access memory (RAM), a cache, or any other storage media in which information is stored for any duration (e.g. for extended period time periods, permanently, brief instances, for temporarily buffering, and/or for caching of the information). Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include any other medium which can be used to store the desired electronic format of information and which can be accessed by the processor or processors or at least a portion of a computing device. In various embodiments, the memory 232 may include an SD memory card, an internal and/or external hard disk, USB memory device, or similar modular memory.

Program code embodied on a computer readable medium may be transmitted using any appropriate medium, including but not limited to wireless, wireline, optical fiber cable, RF, etc., or any suitable combination of the foregoing. Computer program code for carrying out operations for aspects of the present system may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Upon being received at patient monitor 110, the patient data may be processed by local data processing module 226. In particular, local data processing module 226 may include an alarm generation system 228, which may generate alarms based on individual parameters of the patient data, under certain conditions. The alarms may be accompanied by visual health status alerts, which may be displayed in UI 236 and/or other display devices. Alarm generation system 228 may compare values of the patient data with one or more threshold values stored in the memory 232. In some examples, patient monitor 110 may include one or more lookup tables stored in the memory 232 with the one or more threshold values. During processing of the patient data by alarm generation system 228, a threshold value of the one or more threshold values may be retrieved from the one or more lookup tables and compared to a corresponding value of the patient data. For example, if a blood pressure of the patient exceeds a threshold blood pressure (e.g., retrieved from the one or more lookup tables), an alarm may be generated and displayed to a caregiver at a display of the UI 236. In other examples, the threshold values may be entered in to UI 236 by a user when an alarm is configured. The alarm may comprise an audio signal, such as a tone, generated at speaker 238. A clinician may hear the alarm and/or see the health status alert, and may attend to the patient. When the clinician attends to the patient, the clinician may terminate the alarm. The alarm may also be generated at a second location, such as speaker 282 of monitoring station 280. That is, patient monitoring system 200 may be configured such that a first alarm is generated at patient monitor 110 with a first volume/tone/etc., and a second alarm is generated at monitoring station 280 with a second volume/tone/etc.

The conditions under which the alarms are generated may be configured by a user of patient monitor 110, such as a clinician, registered nurse, attending physician, or a different type of caregiver. The user may configure one or more alarms using a UI 229 of alarm generation system 228. UI 229 may be displayed within UI 236 on a display screen of patient monitor 110, for example.

The patient data (e.g., acquired physiological parameters) may be transmitted over the network 272 to server 240. The patient data may additionally or alternatively be processed at server 240. For example, under some conditions, the patient data received at patient monitor 110 from the one or more sensors 270 may be transmitted to server 240 over the network 272 without being processed at local data processing module 226, and the patient data may be processed at a server 240.

In particular, server 240 may include a processor 241 and a multi-condition alarm system 260 stored in a memory 242 of server 240, which may be used to configure a second type of multi-condition alarms. That is, alarms based on single parameters may be configured by a first clinician at alarm generation system 228, via UI 236. The multi-condition alarms may be additionally or alternatively configured by a second clinician using multi-condition alarm system 260 of server 240. The second clinician may be the same as the first clinician, or the second clinician may be different from the first clinician. The second clinician may configure the alarms via a UI 262, as described in greater detail below. The multi-condition alarms may be based on a plurality of conditions that occur concurrently.

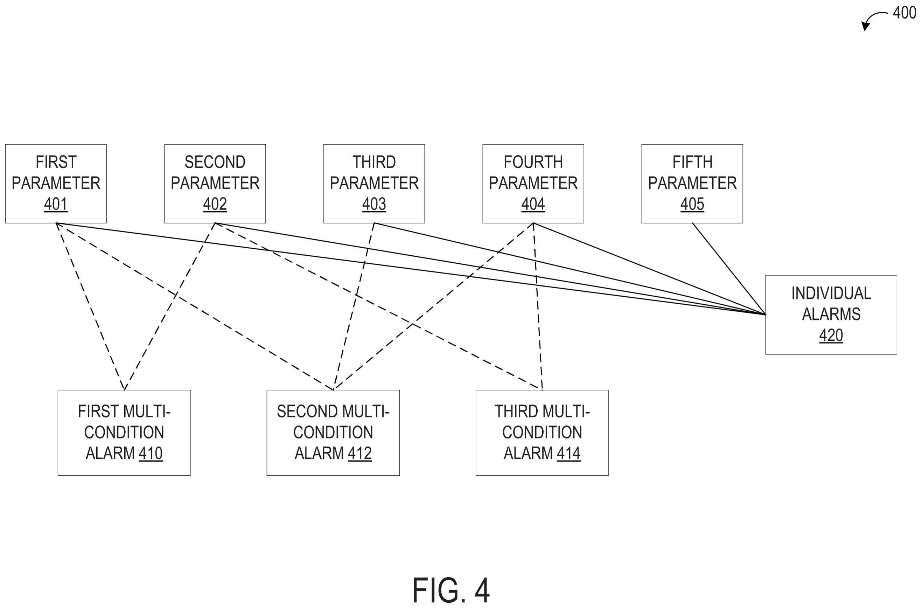

Referring briefly to FIG. 4, a schematic diagram 400 shows an exemplary configuration of conditional alarms that may be configured from various physiological parameters of a patient. Schematic diagram 400 includes five parameters that may be monitored by a patient monitor such as patient monitor 110 of FIG. 1: a first parameter 401, a second parameter 402, a third parameter 403, a fourth parameter 404, and a fifth parameter 405. A set of individual alarms 420 may be configured for parameters 401, 402, 403, 404, and 405 at the patient monitor. That is, a first individual alarm 420 may be generated if first parameter 401 exceeds a first threshold; a second individual alarm 420 may be generated if second parameter 401 exceeds a second threshold; a third individual alarm 420 may be generated if second parameter 401 exceeds a third threshold; and so on. The individual alarms may be outputted at the patient monitor.

Additionally or alternatively, various multi-condition alarms may be configured for a plurality of parameters, including a first multi-condition alarm 410, a second multi-condition alarm 412, and a third multi-condition alarm 414. First multi-condition alarm 410 may be generated in response to a combination of first parameter 401 exceeding a first threshold value and second parameter 402 exceeding a second threshold value. Second multi-condition alarm 412 may be generated in response to a combination of first parameter 401 exceeding a third threshold value, third parameter 403 exceeding a fourth threshold value, and fourth parameter 404 exceeding a fifth threshold value. Third multi-condition alarm 414 may be generated in response to a combination of second parameter 401 exceeding a sixth threshold value and fourth parameter 404 exceeding a seventh threshold value. Each of the first, second, third, fourth, fifth, sixth, and seventh threshold values may be different. The multi-condition alarms 410, 412, and 414 are generated at a multi-condition alarm system, such as multi-condition alarm system 260. The multi-condition alarms may be outputted at various speakers communicatively coupled to the multi-condition alarm system, such as at a nurse's station or a remote edge device. The individual alarms 420 and the multi-condition alarms 410, 412, and 414 may be generated independently. That is, the generation of one of the multi-condition alarms may not affect the generation of any of the other multi-condition alarms or the generation of any individual alarms, and vice versa. One exception may be in the event of paused individual alarms. When individual audio alarms are configured to pause, an active audio alarm may be paused. If a multi-condition audio alarm then occurs, it will sound, overriding the individual audio alarm pause. The priorities of the multi-condition alarms 410, 412, and 414 can also be individually increased or decreased, and one or more tones of the multi-condition alarms 410, 412, and 414 can be individually adjusted, within the guidelines established by relevant standards and regulations.

It should be appreciated that the permitted combinations of the multi-condition alarms 410, 412, and 414 are not limited to logical AND operations, and may include different logical operations (XOR, NOT, etc.). The multi-condition alarms 410, 412, and 414 could also be based on inverted thresholds. For example, a multi-condition alarm may be generated in response to a first parameter being greater than a first threshold value, AND a second parameter being less than a second threshold value.

Returning to FIG. 2, configuring the alarms may include retrieving a template for one or more alarm configurations from an alarm template library 264 stored on server 240. Alarm configurations that are generated by different users via multi-condition alarm system 260 may be stored in alarm template library 264, for future use by the same or other users. Additionally, multi-condition alarm system 260 may include a template recommendation system 266 that may generate recommendations to users of multi-condition alarm system 260 with respect to suitable templates of alarm template library 264 for configuring a desired multi-condition alarm. For such purpose, template recommendation system 266 may include an AI analytics engine 268, which may include one or more AI models that are trained to provide the recommendations. The AI models may include machine learning (ML) or deep learning (DL) models, or statistical models, or rules-based models, or a different kind of model. As an example, AI analytics engine 268 may take as input a plurality of patient parameters that are wished to be monitored, and AI analytics engine 268 may output a template for a multi-condition alarm configuration for monitoring the patient parameters. For example, the template may be recommended by AI analytics engine 268 based on historical patient data, clinician preferences, hospital protocols, and/or other criteria. In some examples, AI-recommended templates may be guided or modified by a clinician based on patient-specific factors, to ensure clinical relevance and safety. The template may include standard or typical thresholds for the plurality of patient parameters, for example, which may be edited or adjusted by the user. By configuring a new multi-condition alarm using the template rather than configuring the new alarm from scratch, an amount of time may be saved in configuring the new alarm and errors in the new alarm configuration may be reduced.

Server 240 may also serve patient data, alerts, multi-condition alarm templates and/or multi-condition alarms generated at multi-condition alarm system 260 to a monitoring station 280, such as a nurse's station of a care unit. A multi-condition alarm received by monitoring station 280 may be outputted at a speaker 282. In various examples, a user of the multi-condition alarm system 260 at monitoring station 280 may view a plurality of active multi-condition alarms, including parameters and thresholds associated with the active multi-condition alarms, on a display device of monitoring station 280.

The patient data, alerts, and/or multi-condition alarms generated at multi-condition alarm system 260 may also be served to a wireless device of a remote caregiver 290, such as a smart phone 140, so that the remote caregiver 290 may view the patient data and/or receive the alerts/alarms. For example, remote caregiver 290 may receive a multi-condition alarm generated by multi-condition alarm system 260 on smart phone 140 when a measured value of the patient data (e.g., acquired by sensors 270) of a patient of remote caregiver 290 exceeds a threshold value configured using multi-condition alarm system 260. Remote caregiver 290 may view a corresponding alert and/or patient data in an alarm configuration app 142 installed on smart phone 140. Alarm configuration app 142 may output the alarm, as well as display clinical data or links to clinical data. In some examples, remote caregiver 290 may configure the multi-condition alarms generated by multi-condition alarm system 260 via alarm configuration app 142. For example, a version of UI 262 may be included in alarm configuration app 142, and remote caregiver 290 may configure the alarms via the version.

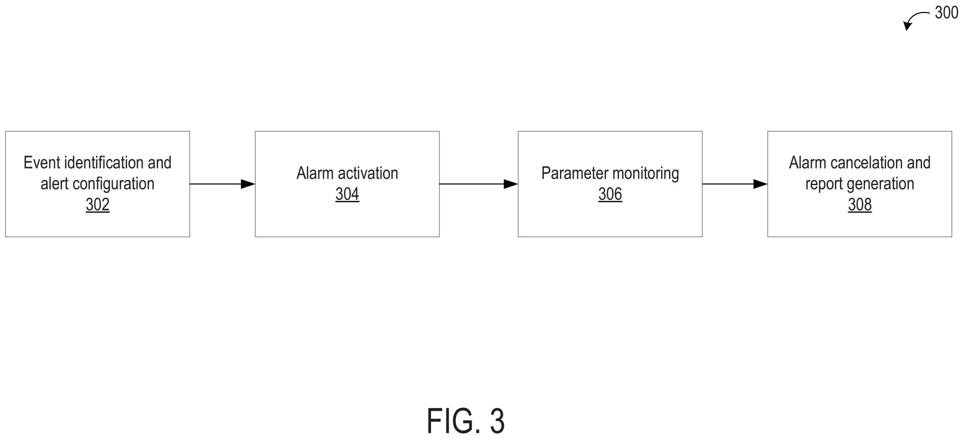

Referring now to FIG. 3, a schematic diagram 300 illustrates a general, high-level procedure for managing the configuration of multi-condition alarms by multi-condition alarm system 260 within patient monitoring system 200 of FIG. 2. The procedure includes four stages.

At a first stage 302, a first user (e.g., a clinician) identifies one or more events that are desired to be detected or that might contribute to detecting a condition of a patient. The clinician may also define thresholds for a set of parameters (e.g., physiological patient data) for each event that are desired to be monitored and considered concurrently. If specified combinations of parameters exceed predefined thresholds, a multi-combination alarm may be generated. The multi-combination alarm may have a different tone or sound than an individual alarm generated by a patient monitor. That is, the clinician may prescribe a type of alarm, meaning, specific audio content that is played at a speaker (e.g., a tone, a message, etc.). The clinician may define the events and thresholds using a GUI of multi-condition alarm system 260, such as UI 262. The first users may identify events and define thresholds for a plurality of alarms for a single patient, or a plurality of patients.

At a second stage 304, a second user of the alarm generation system may configure the alarms based on the clinician's input in stage 302, and activate the alarms. The second user may be a supervisor of the clinician, or the second user may be the same as the first user (e.g., the clinician). The second user may configure the one or more alarms using UI 262, although the second user may access UI 262 (e.g., server 240) via a different computing device than the first user. The alarms may be generated when various conditions or criteria are met in combination, as described above. The second user specifies the types of alarms/alarms that will be generated, and the conditions under which they will be generated, based on the events desired to be monitored by the clinician. In some cases, configuring the alarms may include downloading a template for one or more alarms from a template library (e.g., alarm template library 264 of FIG. 2).

At a third stage 306, each patient monitor of each respective patient continuously monitors the physiological parameters of the patient (e.g., the patient data) in real time. The patient data is transmitted to the multi-condition alarm system in real time. At the multi-condition alarm system, the patient data may be compared to the thresholds set by the first and second clinicians. When conditions are met, meaning, if two or more parameters exceed respective thresholds concurrently, a corresponding alarm is generated for the respective patient. For example, the alarm may be outputted at speaker 282, or a different speaker.

At a fourth stage 308, after the multi-condition alarm is generated, the alarm may be terminated by the clinician, temporarily or permanently, and the multi-condition alarm system logs all events and configurations for future reference and compliance. In some examples, a multi-condition alarm may be terminated when a corresponding event ends. The alarms may also be terminated by either of the clinician or the supervisor. When the alarms are terminated, normal monitoring of the patient data continues. The normal monitoring may include individual alarms at the patient monitors, which retain their normal functionality.

As a number of multi-condition alarms configured for the plurality of patients increases, the first caregiver may experience alarm fatigue, as a result of hearing alarms that are triggered on a regular or frequent basis. To address this, either of the first or second caregivers may configure one or more alarms of the plurality of patients to have different characteristic features, which may allow the alarms to be differentiated. Differentiating the alarms may allow caregivers to focus primarily on certain alarms, while paying less attention to other alarms. For example, one type of alarm (e.g., one tone) may correspond to a patient of the first caregiver, and a second type of alarm may correspond to a patient of a different caregiver. Additionally, either of the first or second caregivers may configure one or more of the previously configured multi-condition alarms to be silenced, as described in greater detail below.

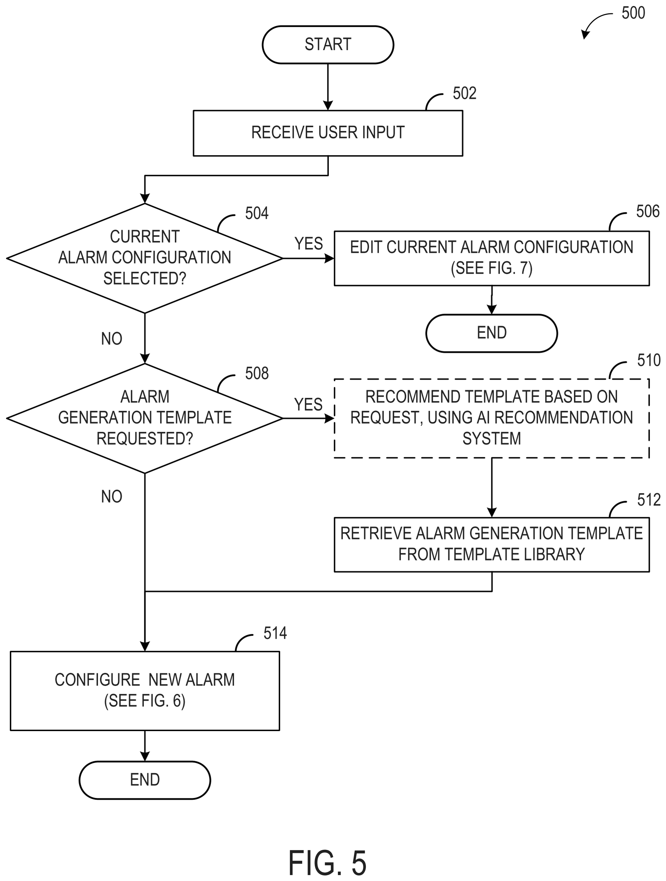

Turning now to FIG. 5, an exemplary high-level method 500 is shown for configuring a multi-condition alarm to be generated in response to a combination of measured patient parameters exceeding corresponding thresholds concurrently. Method 500 may be performed by a multi-condition alarm system, such as multi-condition alarm system 260 of FIG. 2. Operations of method 500 and the other methods described herein may be stored in non-transitory memory of the multi-condition alarm system and executed by a processor of the multi-condition alarm system (e.g., processor 241 of server 240 of FIG. 2).

Method 500 begins at 502, where method 500 includes receiving a user input from a user of the multi-condition alarm system via a GUI of the multi-condition alarm system (e.g., GUI 262). The user input may vary depending on a demand of the user. In some examples, the user input may include a selection of a current multi-condition alarm configuration, which the user wishes to edit or adjust. In other examples, the user input may include a request to configure a new multi-condition alarm.

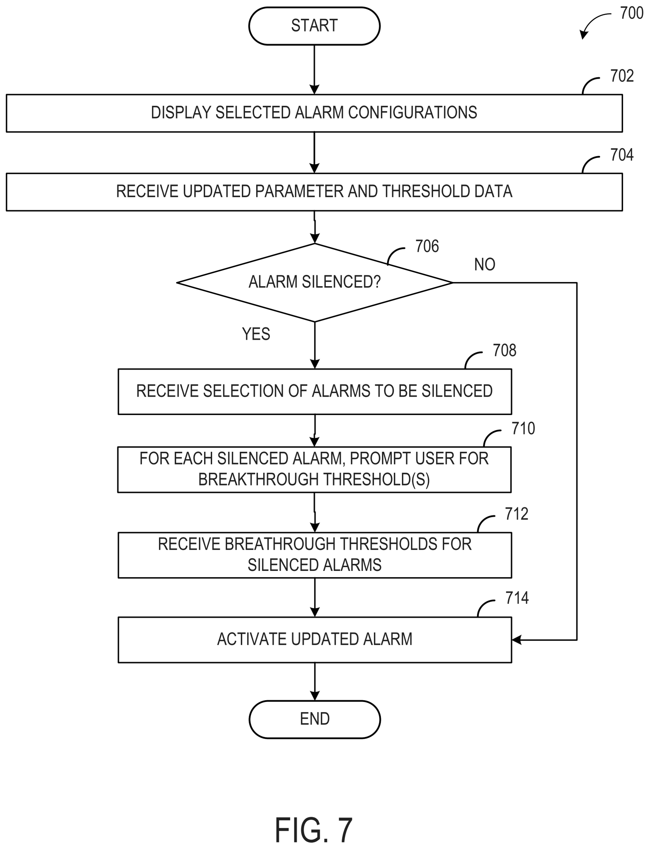

At 504, method 500 includes determining whether a current multi-condition alarm configuration has been selected by the user, which the user wishes to review or edit. If at 504 it is determined that the current alarm configuration has been selected, method 500 proceeds to 506. At 506, method 500 includes editing the multi-condition alarm configuration in accordance with further user inputs. Editing the multi-condition alarm configuration is described below in reference to FIG. 7.

Alternatively, if at 504 it is determined that a current alarm configuration has not been selected by the user, method 500 proceeds to 508. At 508, method 500 includes determining whether an alarm generation template has been requested by the user. The alarm generation template may be one of a plurality of templates for different multi-condition alarm configurations that have been previously created and stored in a centralized template library of the multi-condition alarm system (e.g., alarm template library 264 of FIG. 2).

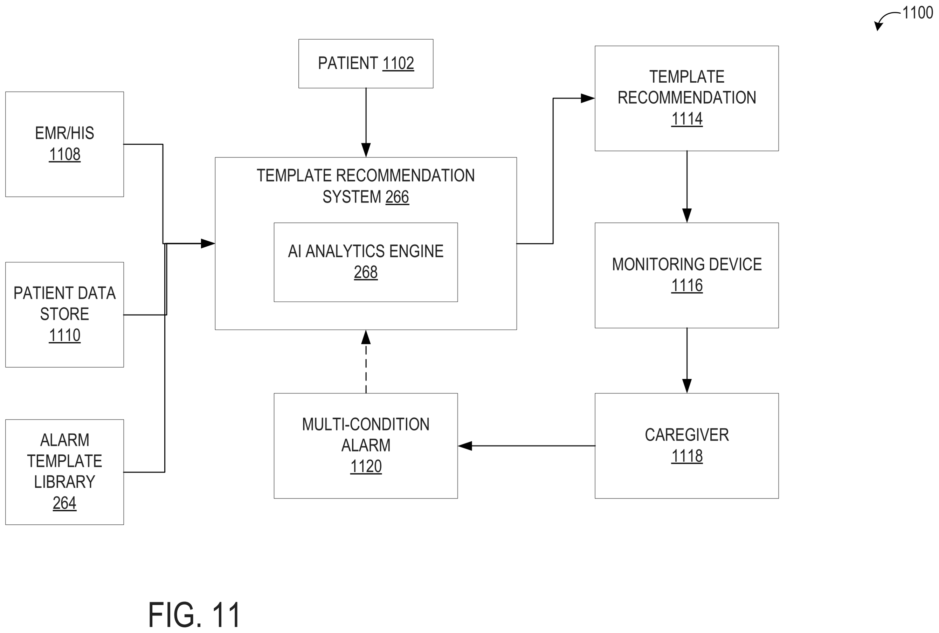

If at 508 it is determined that an alarm generation template has been requested by the user, method 500 proceeds to 510. At 510, method 500 includes optionally recommending a template based on the user request. That is, the user input may specify (e.g., select in the GUI) a template for a multi-condition alarm configuration, or the user input may include a request for a recommendation of a suitable template. In some examples, the template may be recommended by a template recommendation system of the alarm generation system based on an output of an AI model (e.g., AI analytics engine 268). For example, the request for the recommendation may include two or more parameters that the user wishes to monitor, and the template recommendation system may recommend a template of the template library that includes the two or more parameters. The template recommendation system may provide suggestions based on various criteria, such as patient history or type, clinician preference, patient condition, a hospital preference, a global/regional preference, etc. The template recommendation system is described in greater detail below in reference to FIG. 11.

If a specific template is requested by the user, method 500 proceeds to 512, where method 500 includes retrieving the selected template from the template library.

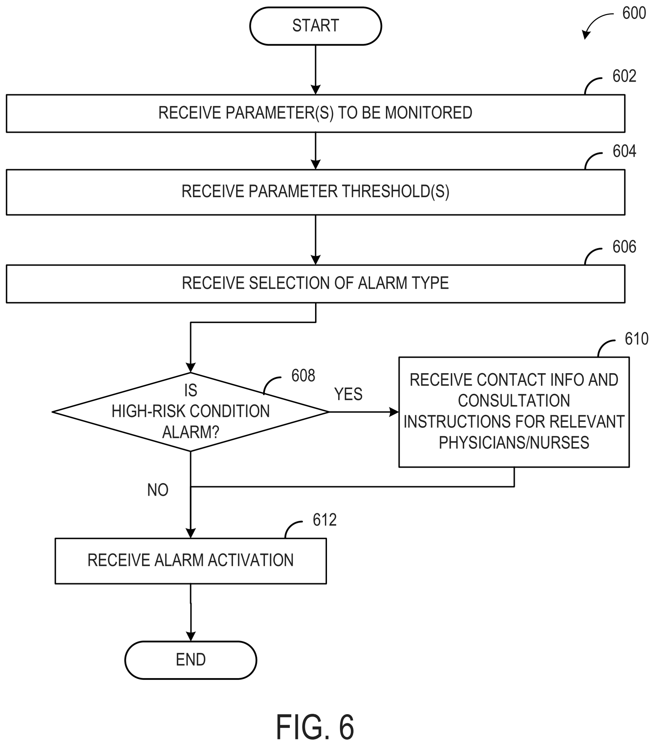

At 514, method 500 includes configuring a new multi-condition alarm in accordance with the user input (which may include a template). Configuring the new multi-condition alarm is described in greater detail below in reference to FIG. 6.

Referring now to FIG. 6, an exemplary method 600 is shown for configuring a new multi-condition alarm using a multi-condition alarm system, such as multi-condition alarm system 260 of FIG. 2. In various examples, method 600 is performed as part of method 500 described above in reference to FIG. 5.

Method 600 begins at 602, where method 600 includes receiving one or more parameters that are desired to be monitored from a user of the multi-condition alarm system. For example, the one or more parameters may be selected in a GUI of the multi-condition alarm system as part of a request by the user to configure a new multi-condition alarm. The parameters may be desired to be monitored in combination. For example, the user may specify a heart rate (HR) as a first parameter, and a blood pressure (BP) as a second parameter. The user may wish to configure the alarm to be generated in response to both of the HR exceeding a first threshold, and the BP exceeding a second threshold.

At 604, method 600 includes receiving threshold values for each of the parameters to be monitored. For example, the user may enter in the threshold values in one or more input fields of the GUI, or may select suitable thresholds value using a control element of the GUI, such as a virtual slider, knob, checkbox, etc. The threshold values may include upper and lower threshold values for two or more parameters. For example, one parameter may be a HR, and the user may specify either or both of an upper HR threshold (e.g., 100 BPM) and a lower HR threshold (e.g., 60 BPM).

Alternatively or additionally, the threshold values may include a threshold rate of change of one or more parameters. The threshold rate of change may be based on a duration over which a specified difference in parameter readings is observed. For example, for a HR parameter, the threshold rate of change may be an amount of time it takes the HR to change by 20 BPM, where if the HR changes by more than 20 BPM in 30 seconds the threshold rate of change is exceeded. If the HR changes by less than 20 BPM in 30 seconds, or if the HR changes by more than 20 BPM over a longer period of time, the threshold rate of change is not exceeded.

At 606, method 600 includes receiving a selection of a type of the multi-condition alarm. The type of the multi-condition alarm may include one of various distinctive audio signals. The various audio signals may include one or more tones, which may be associated with different priorities. For example, a first tone may be a high priority alarm; a second tone may be a medium priority alarm; a third tone may be a low priority alarm; and so on. The audio signals may also incorporate various harmonics to differentiate between different alarms. In some examples, the audio signals may include recorded verbal messages, which may indicate a health concern associated with an alarm. The audio signals may include combinations of tones, harmonics, messages, and/or other types of audio signals. The alarm type may be selected by the user via the GUI, using one or more virtual control elements known in the art.

At 608, method 600 includes determining whether the alarm represents a high-risk condition, meaning a condition where a patient being monitored by the alarm generation system is at a high risk of injury. In the event of a high-risk condition alarm, an attending physician, nurse, or other caregiver of the patient may be notified for immediate consultation. Additionally, the type of alarm may be adjusted, or a volume of the alarm, or a different characteristic of the alarm. In some examples, the user may indicate via the GUI whether the alarm is a high risk alarm. In other examples, the alarm generation system may determine that the alarm is a high risk alarm based on the selected parameters.

If at 608 it is determined that the alarm is a high-risk condition alarm, method 600 proceeds to 610. At 610, method 600 includes receiving contact information and/or consultation instructions directed to the attending physician, nurse, or other caregiver. The contact information may include, for example, a phone number of the caregiver.

At 612, method 600 includes receiving an activation of the multi-condition alarm from the user, and method 600 ends. That is, after the user has specified the various characteristics of the multi-condition alarm, the user may activate the alarm via the GUI. After the alarm is activated, the patient parameters of the alarm may be monitored, and if the parameter thresholds are met, the multi-condition alarm may be generated.

Referring now to FIG. 7, an exemplary method 700 is shown for adjusting a configuration of one or more multi-condition alarms using a multi-condition alarm system, such as multi-condition alarm system 260 of FIG. 2. In particular, method 700 may be performed to facilitate a user selecting and silencing a subset of a number of previously configured multi-condition alarms, while allowing other multi-condition alarms to remain active. In various examples, method 700 is performed as part of method 500 described above in reference to FIG. 5.

Method 700 begins at 702, where method 700 includes displaying the alarm configurations of a selection of active multi-condition alarms generated using the multi-condition alarm system. For example, the one or more multi-condition alarms may correspond to a plurality of patients within a care unit of a hospital. The one or more multi-condition alarms may alert one or more caregivers of the care unit when physiological parameters of a patient of the plurality of patients exceeds threshold values in combination. The configurations of the selected active multi-condition alarms may be displayed in a GUI of the multi-condition alarm system (e.g., GUI 262). For each active multi-condition alarm displayed, the parameters and thresholds considered by the multi-condition alarm may be displayed, so that the user can determine a relative importance of the multi-condition alarm.

In some examples, the multi-condition alarm system may include a predictive algorithm that is trained to indicate or suggest to the user a condition of the patient, based on the parameters being monitored by a multi-condition alarm of the active multi-condition alarms. The predictive algorithm may take as input the parameters and thresholds of the multi-condition alarm, and output one or more possible health conditions of the patient that match the parameters and thresholds. For example, a case of hypertensive patients commonly demonstrating hyperkinetic hypertension at the early course of the disease and tachycardia can be an indicator of established hypertension associated with obesity, metabolic abnormalities and obstructive sleep apnea. The hyperkinetic hypertension and tachycardia may be inputs into the predictive algorithm, and the predictive algorithm may output an indication of possible obesity, metabolic abnormalities, and obstructive sleep apnea.

At 704, method 700 may include receiving updated parameter and threshold data from the user for one or more of the selected active alarms via the GUI. For example, the user may select controls in the GUI to increase or decrease a threshold value for a parameter, or add a new parameter/threshold combination to be monitored.

At 706, method 700 includes determining whether one or more alarms of the active multi-condition alarms are selected to be silenced by the user. That is, the user may select one or more of the alarms via the GUI to be silenced, without affecting other individual or multi-condition alarms. If at 706 it is determined that no alarms are desired to be silenced, method 700 proceeds to 712, where the one or more updated multi-condition alarms are activated. Alternatively, if at 706 it is determined that the user has selected one or more of the configuration of active multi-condition alarms to be silenced, method 700 proceeds to 708.

At 708, method 700 includes receiving a selection of alarms to be silenced from the GUI. Then, at 710, for each alarm to be silenced, method 700 includes prompting the user to optionally specify one or more breakthrough thresholds for the parameters included in the silenced multi-condition alarm configuration. The breakthrough thresholds may be thresholds outside a typical range of thresholds used for an alarm, which may provide an additional measure of precaution. In other words, a clinician may determine that a particular multi-condition alarm should remain silent under normal conditions, where no breakthrough threshold is needed. However, for scenarios where reactivation is desired, a clinician can set breakthrough thresholds where the alarm will be reactivated (at the breakthrough threshold). This flexible approach ensures that alarms remain meaningful, while avoiding undesired alerts.

For example, a multi-condition alarm may be configured to be triggered if an HR of a patient exceeds 100 BPM and a systolic BP of a patient exceeds 120 mmHg. If the multi-condition alarm is silenced, the user may be prompted to optionally enter a breakthrough threshold that if achieved, will generate an alarm. The user may enter a first breakthrough threshold of 110 BPM for the HR, and a second breakthrough threshold of 130 mmHg for the systolic BP. If the HR of the patient exceeds 100 BPM and the systolic BP exceeds 120 mmHg, the alarm will not be generated (e.g., it is silenced). However, if either the HR increases above 110 or the systolic BP increases above 130, the breakthrough alarm will be generated. The breakthrough alarm may have a different alarm type (tone, harmonic, message, etc.) than the multi-condition alarm thresholds.

In this way, the user is not prompted to define breakthrough alarms and/or the breakthrough alarms are not generated unless one or more multi-condition alarms are silenced, and the user is prompted to define the breakthrough alarms and/or the breakthrough alarms are generated in response to the one or more multi-condition alarms being silenced. Thus, a consumption of memory and processing resources entailed by configuring the breakthrough alarms may be advantageously reduced, in comparison with an alternative scenario where the breakthrough alarms are generated for each multi-condition alarm or all alarms.

In some examples, the breakthrough thresholds may be determined automatically and configured by default. If the user does not manually edit the breakthrough thresholds, the default thresholds may be applied. In some examples, the default breakthrough thresholds may be calculated as a percentage over an initial threshold set for an individual parameter. For example, if a multi-condition threshold of 100 BPM is set for an HR parameter for a multi-condition alarm, the breakthrough threshold may be set at 10% over the multi-condition threshold, meaning 110 BPM. In other examples, the default breakthrough thresholds may be established based on clinical guidelines, hospital policies, etc. In some cases, the multi-condition thresholds may be established by a clinician, and the breakthrough thresholds may be set by a supervisor of the clinician.

At 712, method 700 includes receiving the breakthrough thresholds for the silenced alarms from the GUI, and at 714, method 700 includes activating the updated alarm. Method 700 ends.

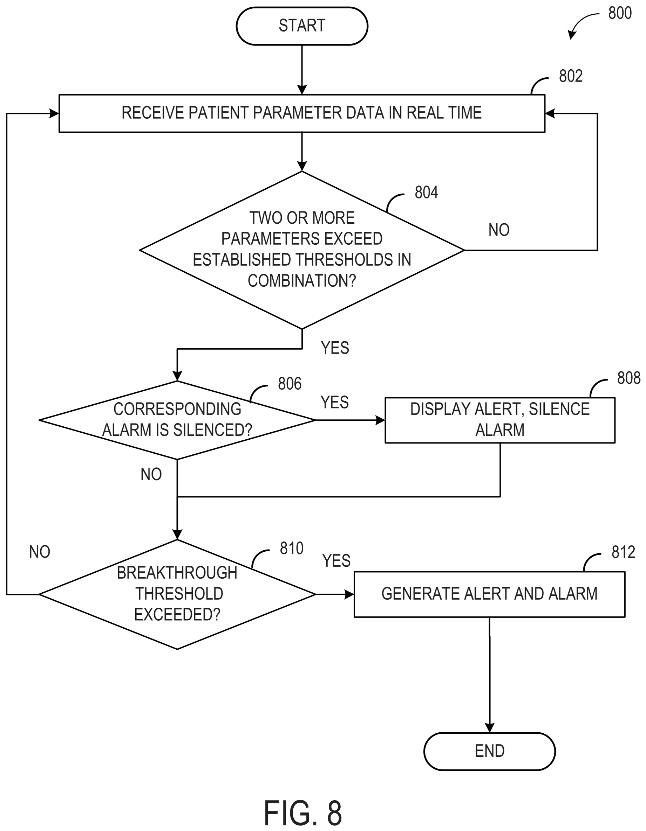

Turning now to FIG. 8, an exemplary method 800 is shown for generating a multi-condition alarm configured using a multi-condition alarm system such as multi-condition alarm system 260 of FIG. 2. Method 700 may be performed by a processor of the multi-condition alarm system.

Method 800 starts at 802, where method 800 includes receiving physiological patient parameter data in real time, from one or more sensors arranged on a body of the patient (e.g., sensors 270). The parameter data (e.g., measurements or readings) may be continuously received at the patient monitor, and transmitted to the multi-condition alarm system via a network (e.g., network 272).

At 804, method 800 includes determining whether two or more measured parameters of the parameter data exceed thresholds established for the measured parameters. The thresholds may be defined using the multi-condition alarm system as described above in reference to FIGS. 6 and 7.

If at 804 it is determined that two or more measured parameters of the parameter data exceed the thresholds established for the measured parameters, method 800 proceeds to 806. At 806, method 800 includes determining whether a multi-condition alarm configured based on the measured parameters and respective thresholds has been silenced, as described above in reference to FIG. 7. If the multi-condition alarm is silenced, method 800 proceeds to 808, where method 800 includes displaying an alert associated with the alarm on a display device communicatively coupled to the multi-condition alarm system, such as a display device of a nurse's station overseeing a care unit of the patient. However, no multi-condition alarm is generated, and method 800 proceeds to 810.

Alternatively, if the alarm is not silenced in accordance with method 700, method 800 proceeds to 810. At 810, method 800 includes determining whether a breakthrough threshold has been exceeded. The breakthrough threshold may be an additional threshold of a parameter defined in accordance with method 700. For example, the breakthrough threshold may be a higher threshold than an original threshold established for the parameter.

If the breakthrough threshold has been exceeded at 810, method 800 proceeds to 812, where method 800 includes generating both of the alert and the alarm. The alarm may be generated at a speaker of a monitoring station, such as monitoring station 280. Additionally or alternatively, both of the alert and the multi-condition alarm may be generated at an additional location, such as a remote edge device. The alarm may continue to be generated until the alarm is terminated (e.g., switched off or cancelled) by a caregiver.

If the breakthrough threshold is not exceeded at 810, method 800 proceeds back to 802, and the parameter data of the patient continues to be received and monitored in real time, and method 800 ends.

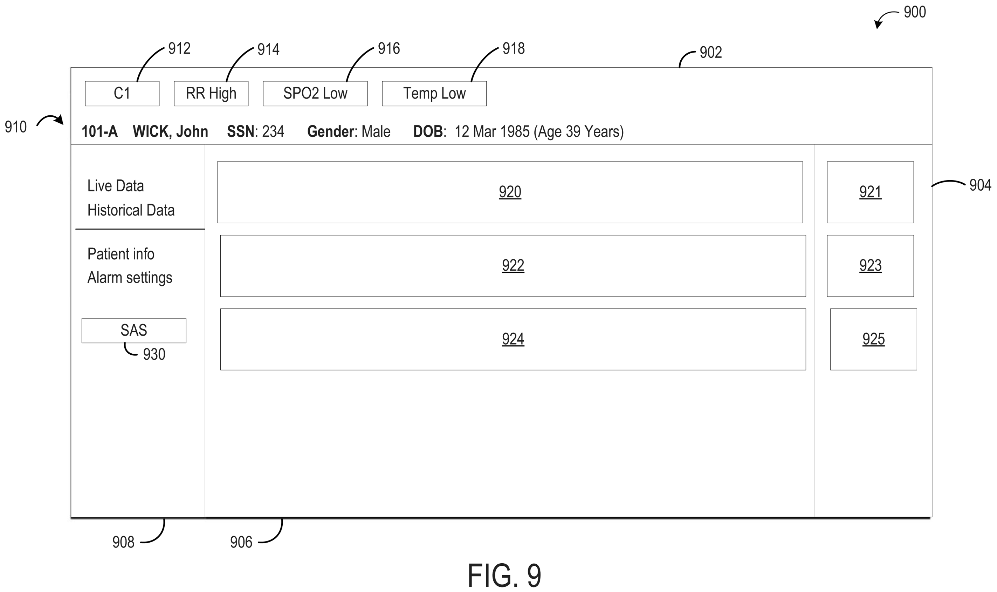

FIG. 9 shows an exemplary GUI 900 of a patient monitoring system, such as patient monitoring system 200 of FIG. 2. GUI 900 may display physiological patient data of a single patient acquired in real time via a patient monitor, such as patient monitor 110 of FIG. 1. For example, GUI 900 may be displayed at a central viewing station of a care unit of a hospital, such as a nurses' station. GUI 900 or a GUI with similar features may also be displayed at the patient monitor. In some examples, GUI 900 may be displayed by selecting a control element of a more general GUI of the patient monitoring system that shows condensed information of a plurality of patients.

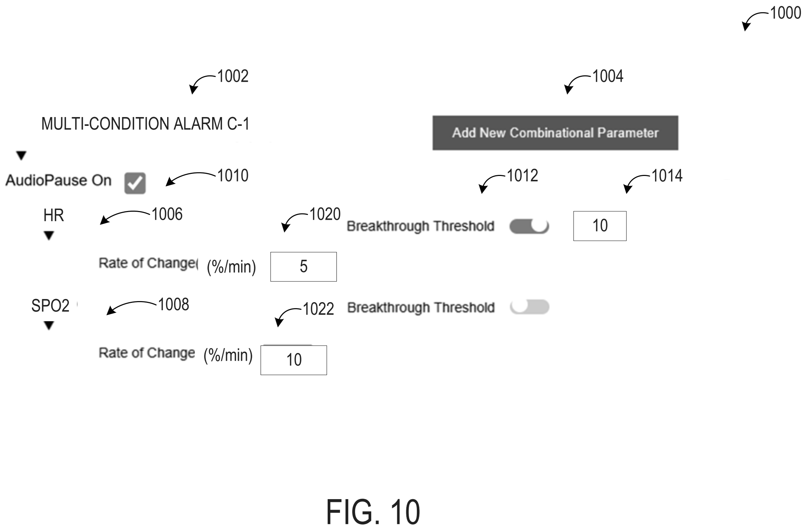

In the depicted embodiment, GUI 900 includes a top display panel 902, a right display panel 904, a central display panel 906, and a left display panel 908. Top display panel 902 may include patient information 910, such as an identification code or number of the patient, a name of the patient, and/or other information such as a social security number (SSN), gender, data of birth (DOB), etc. Top display panel 902 may also show an indication of one or more alarms and/or alerts that have been set for the patient. The one or more alarms may include multi-condition alarms and individual alarms/alerts. In the depicted embodiment, four alarms are indicated: a multi-condition alarm 912 (C1), a respiratory rate (RR) alarm 914, an SPO2 alarm 916, and a temperature alarm 918. RR alarm 914, SPO2 alarm 916, and temperature alarm 918 are individual alarms, where an alarm is generated when a respective parameter (RR, SPO2, or temperature) is greater than or less than a threshold value set by a caregiver. For example, in the depicted embodiment, RR alarm 914 may be generated when an RR of the patient exceeds a threshold RR; SPO2 alarm 916 may be generated when an SPO2 of the patient decreases below a threshold SPO2; and temperature alarm 918 may be generated when a temperature of the patient decreases below a threshold temperature. Multi-condition alarm 912 may be generated when various parameters exceed respective thresholds, as described above. Multi-condition alarm 912 is described in greater detail below in reference to FIG. 10. In various examples, the graphical elements displayed in GUI 900 to indicate multi-condition alarm 912, RR alarm 914, SPO2 alarm 916, and temperature alarm 918 may be selectable elements, where a user can select a respective alarm and view information about the respective alarm, for example, in a GUI similar to GUI 1000 of FIG. 10 described below. The graphical elements may also include an indication of whether a respective alarm has been silenced.

It should be appreciated that the components and the organization of the components in GUI 900 are described herein for illustrative purposes, and different components may be included in GUI 900, or organized differently, without departing from the scope of this disclosure.

Central display panel 906 may show physiological patient data in real time, which may be displayed as separate components corresponding to different parameters of the physiological patient data. For example, a first component 920 may show a first graphical display of the RR of the patient. A second component 922 may show a second graphical display of an electrocardiograph (ECG) readout of the patient. A third component 924 may show a third graphical display indicating the SPO2 of the patient; and so on. For each of the components displayed in central display panel 906, specific information about the respective parameter value may be displayed in a corresponding component of right display panel 904. For example, a current RR and the threshold RR of the patient may be shown in a first informational component 921; a current heart rate (HR) of the patient may be shown in a second informational component 923; a current SPO2 and the threshold SPO2 of the patient may be shown in a third informational component 925; and so on.

Left display panel 908 may include additional information about the patient and/or control elements for adjusting a display of data in GUI 900. For example, the user may select to view live data or historical data in central display panel 906 and right display panel 904. The user may also view additional patient data, alarm settings, or other relevant data, depending on an implementation.

Left display panel 908 also includes a selective alarm silencing (SAS) control element 930 (e.g., a button), which when selected, may display a GUI that allows the user to selectively silence one or more of multi-condition alarm 912, RR alarm 914, SPO2 alarm 916, and temperature alarm 918. An example of the GUI is shown in FIG. 10.

FIG. 10 shows an exemplary GUI 1000 of a multi-condition alarm system, referred to herein as the system, such as multi-condition alarm system 260 of FIG. 2. GUI 1000 may be generated as a result of selecting a selective alarm silencing control element of a GUI such as SAS control element 930 of GUI 900 described above. GUI 1000 may allow a user of the system to create, adjust, or silence one or more multi-condition alarms, such as multi-condition alarms 410, 412, and/or 414. In the depicted example of GUI 1000, a multi-condition alarm 1002 is shown for an exemplary patient, which may be the same as or similar to the multi-condition alarm 912 of FIG. 9 above. Multi-condition alarm 1002 includes a first heart rate (HR) parameter 1006 and a second blood oxygen (SPO2) parameter 1008. If desired, the user (e.g., a caregiver) can add an additional parameter for multi-condition alarm 1002 via a button 1004. In other embodiments, individual alarms may also be displayed in GUI 1000.

In the depicted example, HR parameter 1006 and SPO2 parameter 1008 are rate of change parameters, where multi-condition alarm 1002 is generated when a first rate of change of HR parameter 1006 exceeds a first rate of change threshold (e.g., 5% change in BPM in a minute) AND a second rate of change of SPO2 parameter 1008 exceeds a second rate of change threshold (e.g., 10% change in a minute). The first rate of change threshold may be entered into a first rate of change field 1020 by the user, and the second rate of change threshold may be entered into a second rate of change field 1022 by the user. If the first rate of change of HR parameter 1006 does not exceed the first rate of change threshold OR the second rate of change of SPO2 parameter 1008 does not exceed the second rate of change threshold, multi-condition alarm 1002 is not generated. In other examples, parameter thresholds may be used rather than rate of change thresholds, as described herein.

Multi-condition alarm 1002 can be silenced by the user as described in method 700 of FIG. 7 by selecting an AudioPause On control 1010. When AudioPause On control 1010 is selected, multi-condition alarm 1002 will not be generated. When AudioPause On control 1010 is selected, the user may be prompted to optionally set a breakthrough threshold, above or below which the alarm will be generated despite being silenced via the AudioPause On control 1010. For each parameter, GUI 1000 displays a breakthrough threshold control element 1012 and a breakthrough parameter field 1014. The user may set a breakthrough threshold for HR parameter 1006 by selecting breakthrough threshold control element 1012, and entering in a parameter for the breakthrough control via breakthrough parameter field 1014. In the depicted example, a breakthrough threshold of 10 is established, meaning, if the HR of the patient exceeds 5 % per minute, multi-condition alarm 1002 will remain silenced, but if the HR of the patient exceeds 10% per minute, multi-condition alarm 1002 will be generated.

By using GUI 1000, the user can customize multi-condition alarm 1002 to be generated when various desired criteria are concurrently met, and selectively silence multi-condition alarm 1002 if desired, for example, due to a high number of alarms being activated for the patient. As a precaution, the user can set the breakthrough thresholds to ensure that a parameter does not go unnoticed in the event of a worsening of a health condition of the patient when multi-condition alarm 1002 is silenced. In various examples, GUI 1000 may be integrated into, or may be generated as a pop-up display, of a UI used to monitor the HR, SPO2, and/or other parameters visually at a patient monitor or networked device. Additionally, GUI 1000 may allow the user to quickly and efficiently silence a plurality of alarms of different types displayed in GUI 1000.

FIG. 11 shows a schematic data flow diagram 1100 that shows an exemplary flow of data during template recommendations performed by template recommendation system 266, which may recommend a template for a multi-condition alarm for a patient 1102 to a caregiver 1118. Caregiver 1118 may enter a patient identifier of patient 1102 into template recommendation system, and based on the patient identifier, template recommendation system 266 may retrieve data of the patient from an electronic medical record (EMR) or hospital information system (HIS) 1108. The data may include, for example, patient history, demographic data, lab data, and the like. Template recommendation system 266 may additionally retrieve data from a patient data store 1110, which may include historical physiological data acquired from patient 1102. The data retrieved from EMR/HIS 1108 and patient data store 1110 may be inputted into AI analytics engine 268, which may include one or more AI models trained to recommend a multi-condition alarm template of alarm template library 264. AI analytics engine 268 may output a template recommendation 1114 for patient 1102. Template recommendation system 266 may transmit template recommendation 1114 to a monitoring device 1116, such as monitoring station 280, patient monitor 110, or a different edge device. Caregiver 1118 may view template recommendation 1114 on monitoring device 1116, and may select template recommendation 1114 to generate a multi-condition alarm 1120 based on template recommendation 1114. Caregiver 1118 may edit one or more aspects of multi-condition alarm 1120. For example, caregiver 1118 may add or remove a parameter to be monitored, adjust one or more parameter thresholds of multi-condition alarm 1120, and/or adjust an audio signal associated with multi-condition alarm 1120. In some examples, multi-condition alarm 1120 may be inputted into template recommendation system 266, and template recommendation system 266 may confirm a suitability of multi-condition alarm 1120 for patient 1102. If the suitability is confirmed, a new template may be generated for multi-condition alarm 1120, which may be stored at alarm template library 264 for future use. In this way, customized multi-condition alarms may be quickly and easily generated by caregiver 1118 based on pre-approved templates.

AI analytics engine 268 may interact with alarm template library 264 in several ways. AI analytics engine 268 may help a caregiver create new templates in alarm template library 264 by suggesting multi-conditional parameters for a certain clinical condition the caregiver is concerned with. AI analytics engine 268 could also indicate how templates could vary depending on patient data. AI analytics engine 268 may also suggest to the caregiver appropriate templates from alarm template library 264, based on patient data or a clinical condition indicated by the caregiver. Further, AI analytics engine 268 may generate insights with respect to a template created based on caregiver input without using AI analytics engine 268, for example, with respect to a condition the caregiver wishes to monitor. The insights may include different parameters or threshold values to include in the template, for example.

Further, in some examples, third-party AI/ML applications and/or analytics engines owned by other hospitals may be leveraged by template recommendation system 266 for creating personalized multi-condition alarm settings. The third-party AI/ML applications and/or analytics engines may access alarm template library 264, or other alarm template libraries to generate appropriate template recommendations 1114.

Additionally, or alternatively, caregiver 1118 may browse alarm template library 264 and select a suitable template for patient 1102. In one example, caregiver 1118 may enter in a text query regarding a desired template. For example, caregiver 1118 may type into a UI of the multi-condition alarm system a query such as, "Can you suggest a suitable alarm template for a 50-year old male patient who has undergone cardiac surgery, has a history of diabetes, and is using a pacemaker?". Template recommendation system 266 may use natural language processing to parse the query, and may convert the query into structured data, such as in a JavaScript Object Notation (JSON) format. The structured data query may be processed by template recommendation system 266. Template recommendation system 266 may extract data from the structured data query and input the extracted data into AI analytics engine 268, and AI analytics engine 268 may output a template recommendation 1114.

Thus, the proposed system allows caregivers to configure multi-condition alarms at a centralized location in a hospital, as an alternative to conventional individual parameter alarms typically configured at a patient monitor. The multi-condition alarms are configured to output an audio signal when two or more physiological parameters exceed separate threshold values concurrently. Thus, the conditions for the multi-condition alarms being generated are defined more narrowly than the individual parameter alarms. Said another way, a multi-condition alarm may replace two or more individual parameter alarms. For example, a first individual parameter alarm may be configured to be generated when an HR of a patient exceeds a first threshold, and a second individual parameter alarm may be configured to be generated when a BP of the patient exceeds a second threshold. However, a multi-condition alarm may be configured to be generated when both the HR exceeds the first threshold and the BP exceeds the second threshold. Because trends in the BP and the HR may be related, the multi-condition alarm may replace both of the first and second individual parameter alarms.

Therefore, by providing the multi-condition alarms, an overall number of alarms configured in a patient monitoring system may be advantageously reduced, as caregivers replace at least some of the individual alarms with multi-condition alarms. Thus, the system supports both single and multiple criteria, while ensuring clinician oversight for final decision-making. A template recommendation system may base template recommendations on at least one or a combination of patient history, clinician preferences, hospital policies, or AI-driven analytics, with final approval or modifications guided by the caregiver.

As a technical effect of the proposed system, by reducing the overall number of alarms, a consumption of memory and processing resources of a centralized alarm generation system, and/or a plurality of patient monitors configured to generate alarms, may be reduced. An additional technical effect of the proposed system is that the breakthrough alarms are not defined or generated unless one or more multi-condition alarms are silenced, and no breakthrough alarms may be defined or generated for multi-condition alarms that are not silenced, where a consumption of memory and processing resources entailed by configuring the breakthrough alarms may be advantageously reduced, in comparison with an alternative scenario where the breakthrough alarms are generated for each multi-condition alarm.

Further, the proposed system allows a user to additionally select, from a set of multi-condition alarms defined based on combinations of parameters, or combinations of combinations of parameters, a first subset of the alarms that will be generated when their corresponding conditions are met, and a second subset of the alarms that will be silenced, and not generated when their corresponding conditions are met. Unlike the individual parameter alarms, which may be individually silenced at a respective patient monitor, the multi-condition alarms may be more flexibly controlled from a central location. By allowing some alarms to be selectively silenced, for example, when a caregiver knows that the alarms do not indicate a concerning patient condition, a total number of alarms that are generated in a given care unit may be reduced, which may lessen an alarm fatigue suffered by caregivers in the care unit.

Additionally, the proposed system allows the caregivers to assign a wider variety of tones, sounds, harmonics, messages, etc. to different alarms than is currently supported by existing systems, thereby increasing a differentiation of alarms within the care unit. Different alarm sounds may be configured for different health conditions, caregivers, priorities, and/or other criteria. By increasing the differentiation, caregivers may learn to selectively attend to certain alarms while devoting less attention to other types of alarms, which may further reduce the alarm fatigue.

The disclosure also provides support for a multi-condition alarm system for a patient monitoring system, comprising a processor and a memory storing instructions that when executed, cause the processor to: receive physiological parameters of a plurality of patients via a respective plurality of patient monitors coupled to the plurality of patients, generate a plurality of multi-condition alarms for the plurality of patients, each multi-condition alarm configured to output a respective audio signal when two or more physiological parameters of a respective patient exceed predefined thresholds concurrently, in response to an input from a user of the multi-condition alarm system, configure a first portion of the plurality of multi-condition alarms to be silenced when two or more physiological parameters of a respective patient exceed predefined thresholds concurrently, and configure a second portion of the plurality of multi-condition alarms not to be silenced when two or more physiological parameters of a respective patient exceed predefined thresholds concurrently, in a first condition where two or more physiological parameters of a first multi-condition alarm of the first portion of multi-condition alarms exceed respective predefined thresholds concurrently, not output the respective audio signal, and in a second condition where two or more physiological parameters of a second multi-condition alarm of the second portion of multi-condition alarms exceed respective predefined thresholds concurrently, output the respective audio signal. In a first example of the system, the predefined thresholds are defined by a user of the multi-condition alarm system at a time of configuring the multi-condition alarms. In a second example of the system, optionally including the first example, the plurality of multi-condition alarms are all displayed in a user interface (UI) of the multi-condition alarm system on a display device, such that a user of the multi-condition alarm system may view the physiological parameters and predefined thresholds of each multi-condition alarm of the plurality of multi-condition alarms. In a third example of the system, optionally including one or both of the first and second examples, further instructions are stored in the memory that when executed, cause the processor to: define one or more respective breakthrough thresholds for one or more physiological parameters of a multi-condition alarm of the first portion of multi-condition alarms, the one or more respective breakthrough thresholds configured to generate a second audio signal when a physiological parameter of the one or more physiological parameters exceeds a respective breakthrough threshold. In a fourth example of the system, optionally including one or more or each of the first through third examples, a respective breakthrough threshold of a physiological parameter is calculated automatically as a percentage over a respective predefined threshold of the physiological parameter. In a fifth example of the system, optionally including one or more or each of the first through fourth examples, a respective breakthrough threshold of a physiological parameter is calculated automatically based on clinical guidelines and/or policies of a hospital including the patient monitoring system. In a sixth example of the system, optionally including one or more or each of the first through fifth examples, a respective breakthrough threshold of a physiological parameter is defined based on an input from a user of the multi-condition alarm system via the UI of the multi-condition alarm system. In a seventh example of the system, optionally including one or more or each of the first through sixth examples, a multi-condition alarm is configured by the user based on an alarm generation template requested by the user and retrieved from a template library stored in the memory. In an eighth example of the system, optionally including one or more or each of the first through seventh examples, further instructions are stored in the memory that when executed, cause the processor to: receive a configuration of a multi-condition alarm from the user, and store the configuration as a new template in the template library. In a ninth example of the system, optionally including one or more or each of the first through eighth examples, a copy of one or more templates of the template library are stored at a patient monitor of the plurality of patient monitors or a bedside device communicatively coupled to the patient monitor, and periodically updated from the template library by the multi-condition alarm system. In a tenth example of the system, optionally including one or more or each of the first through ninth examples, the retrieved template is selected from the template library as a result of a recommendation by a template recommendation system of the multi-condition alarm system, based on at least one of: a patient history or patient type, a clinician preference, a patient condition, a hospital preference, and a global or regional preference. In an eleventh example of the system, optionally including one or more or each of the first through tenth examples, the retrieved template is selected by the template recommendation system based on an artificial intelligence (AI) model that takes as input a plurality of patient parameters that are wished to be monitored, and outputs a recommended template of the template library. In a twelfth example of the system, optionally including one or more or each of the first through eleventh examples, a predefined threshold of a multi-condition alarm of the plurality of multi-condition alarms is a rate of change threshold, where the multi-condition alarm is configured to generate the audio signal when a rate of change of a respective physiological parameter exceeds a threshold rate of change.

The disclosure also provides support for a method for a multi-condition alarm system of a patient monitoring system, the method comprising: receiving physiological parameters of a plurality of patients via a respective plurality of patient monitors coupled to the plurality of patients, generating a plurality of multi-condition alarms for the plurality of patients, each multi-condition alarm configured to output a respective audio signal when two or more physiological parameters of a respective patient exceed predefined thresholds concurrently, in response to an input from a user of the multi-condition alarm system, configuring a first portion of the plurality of multi-condition alarms to be silenced when two or more physiological parameters of a respective patient exceed predefined thresholds concurrently, and configuring a second portion of the plurality of multi-condition alarms not to be silenced when two or more physiological parameters of a respective patient exceed predefined thresholds concurrently, in a first condition where two or more physiological parameters of a first multi-condition alarm of the first portion of multi-condition alarms exceed respective predefined thresholds concurrently, not output the respective audio signal, and in a second condition where two or more physiological parameters of a second multi-condition alarm of the second portion of multi-condition alarms exceed respective predefined thresholds concurrently, output the respective audio signal. In a first example of the method, the method further comprises: defining one or more respective breakthrough thresholds for one or more physiological parameters of a multi-condition alarm of the first portion of multi-condition alarms, the one or more respective breakthrough thresholds configured to generate a second audio signal when a physiological parameter of the one or more physiological parameters exceeds a respective breakthrough threshold. In a second example of the method, optionally including the first example, the method further comprises: receiving, at a user interface (UI) of the multi-condition alarm system, a user request for a template of a multi-condition alarm from a template library stored in a memory of the multi-condition alarm system, inputting data of the user request into a template recommendation system of the multi-condition alarm system, and receiving a recommended template as an output of the template recommendation system, retrieving the recommended template from the template library and displaying the recommended template in the UI, receiving a configuration of the multi-condition alarm from the user, based on the recommended template, and activating the multi-condition alarm based on the configuration. In a third example of the method, optionally including one or both of the first and second examples, the template recommendation system recommends the recommended template based on at least one of: a patient history or patient type, a clinician preference, a patient condition, a hospital preference, a global or regional preference, and an output of an artificial intelligence (AI) model that takes as input a plurality of patient parameters that are wished to be monitored, and outputs the recommended template.

The disclosure also provides support for a patient monitoring system, comprising: a plurality of patient monitors, each patient monitor of the plurality of patient monitors configured to monitor physiological parameters of a respective plurality of patients, and a multi-condition alarm system communicatively coupled to the plurality of patient monitors and configured to generate multi-condition alarms when conditions are met, each multi-condition alarm configured to output a respective audio signal when the conditions are met, the conditions including two or more physiological parameters of a patient of the plurality of patients exceeding predefined thresholds concurrently, wherein the multi-condition alarm system further includes a processor and a memory storing instructions that when executed, cause the processor to: in response to a user input, configure a first portion of the multi-condition alarms to be selectively silenced when the conditions are met, while allowing a second portion of the multi-condition alarms to be generated when the conditions are met, in a first condition where the conditions of a first multi-condition alarm of the first portion of multi-condition alarms are met, not output the respective audio signal, and in a second condition where the conditions of a second multi-condition alarm of the second portion of multi-condition alarms are met, output the respective audio signal. In a first example of the system, further instructions are stored in the memory that when executed, cause the processor to: define one or more respective breakthrough thresholds for one or more physiological parameters of a multi-condition alarm of the first portion of multi-condition alarms, the one or more respective breakthrough thresholds configured to generate a second audio signal when a physiological parameter of the one or more physiological parameters exceeds a respective breakthrough threshold, the respective breakthrough threshold either defined based on an input from a user of the multi-condition alarm system via a UI of the multi-condition alarm system, or calculated automatically based on clinical guidelines and/or policies of a hospital including the patient monitoring system or as a percentage over a respective predefined threshold of the physiological parameter. In a second example of the system, optionally including the first example, further instructions are stored in the memory that when executed, cause the processor to: receive, via the UI of the multi-condition alarm system, a user request for a template of a multi-condition alarm from a template library stored in the memory, input data of the user request into a template recommendation system of the multi-condition alarm system, and receive a recommended template as an output of the template recommendation system, retrieve the recommended template from the template library and display the recommended template in the UI, receive a configuration of the multi-condition alarm from the user, based on the recommended template, and activate the multi-condition alarm based on the configuration.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A multi-condition alarm system (260) for a patient monitoring system (100), comprising a processor (241) and a memory (242) storing instructions that when executed, cause the processor to:
receive physiological parameters of a plurality of patients via a respective plurality of patient monitors (110) coupled to the plurality of patients;
generate a plurality of multi-condition alarms (410, 412, 414) for the plurality of patients, each multi-condition alarm (410, 412, 414) configured to output a respective audio signal when two or more physiological parameters of a respective patient exceed predefined thresholds concurrently;
in response to an input from a user of the multi-condition alarm system (260), configure a first portion of the plurality of multi-condition alarms (410, 412, 414) to be silenced when two or more physiological parameters of a respective patient exceed predefined thresholds concurrently, and configure a second portion of the plurality of multi-condition alarms (410, 412, 414) not to be silenced when two or more physiological parameters of a respective patient exceed predefined thresholds concurrently;
in a first condition where two or more physiological parameters of a first multi-condition alarm of the first portion of multi-condition alarms (410, 412, 414) exceed respective predefined thresholds concurrently, not output the respective audio signal; and
in a second condition where two or more physiological parameters of a second multi-condition alarm of the second portion of multi-condition alarms (410, 412, 414) exceed respective predefined thresholds concurrently, output the respective audio signal.

2. The multi-condition alarm system (260) of claim 1, wherein the predefined thresholds are defined by a user of the multi-condition alarm system (260) at a time of configuring the multi-condition alarms (410, 412, 414).

3. The multi-condition alarm system (260) of claim 1, further comprising a user interface (UI) (262);
wherein the memory (242) includes further instructions that, when executed, cause the processor (241) to:
receive physiological parameters and predefined thresholds of each multi-condition alarm of the plurality of multi-condition alarms (410, 412, 414); and
display the physiological parameters and predefined thresholds of each multi-condition alarm on a display device via the UI (262).

4. The multi-condition alarm system (260) of claim 3, wherein further instructions are stored in the memory (242) that when executed, cause the processor (241) to:
during a first scenario where the first portion of the plurality of multi-condition alarms (410, 412, 414) is configured to be silenced, prompt the user to define one or more respective breakthrough thresholds for one or more physiological parameters of a multi-condition alarm of the first portion of multi-condition alarms (410, 412, 414), the one or more respective breakthrough thresholds configured to generate a second audio signal when a physiological parameter of the one or more physiological parameters exceeds a respective breakthrough threshold; and
during a second scenario where no multi-condition alarms are configured to be silenced, not prompt the user to define the one or more respective breakthrough thresholds.

5. The multi-condition alarm system (260) of claim 4, wherein a respective breakthrough threshold of a physiological parameter is calculated automatically as a percentage over a respective predefined threshold of the physiological parameter.

6. The multi-condition alarm system (260) of claim 4, wherein a respective breakthrough threshold of a physiological parameter is calculated automatically based on clinical guidelines and/or policies of a hospital including the patient monitoring system (200).

7. The multi-condition alarm system (260) of claim 4, wherein a respective breakthrough threshold of a physiological parameter is defined based on an input from a user of the multi-condition alarm system (260) via the UI of the multi-condition alarm system (260).

8. The multi-condition alarm system (260) of claim 2, wherein a multi-condition alarm (410, 412, 414) is configured by the user based on an alarm generation template requested by the user and retrieved from a template library (264) stored in the memory (242).

9. The multi-condition alarm system (260) of claim 8, wherein further instructions are stored in the memory (242) that when executed, cause the processor (241) to:
receive a configuration of a multi-condition alarm (410, 412, 414) from the user, and store the configuration as a new template in the template library (264).

10. The multi-condition alarm system (260) of claim 8, wherein a copy of one or more templates of the template library (264) are stored at a patient monitor (110) of the plurality of patient monitors or a bedside device communicatively coupled to the patient monitor (110), and periodically updated from the template library (264) by the multi-condition alarm system (260).

11. The multi-condition alarm system (260) of claim 8, wherein the retrieved template is selected from the template library (264) as a result of a recommendation by a template recommendation system (266) of the multi-condition alarm system (260), based on at least one of:
a patient history or patient type;
a clinician preference;
a patient condition;
a hospital preference; and
a global or regional preference.

12. The multi-condition alarm system (260) of claim 11, wherein the retrieved template is selected by the template recommendation system (266) based on an artificial intelligence (AI) model that takes as input a plurality of patient parameters that are wished to be monitored, and outputs a recommended template of the template library (266).

13. The multi-condition alarm system (260) of claim 1, wherein a predefined threshold of a multi-condition alarm of the plurality of multi-condition alarms (410, 412, 414) is a rate of change threshold, where the multi-condition alarm is configured to generate the respective audio signal when a rate of change of a respective physiological parameter exceeds a threshold rate of change.

14. A method for a multi-condition alarm system of a patient monitoring system, the method comprising:
receiving physiological parameters of a plurality of patients via a respective plurality of patient monitors coupled to the plurality of patients (602, 802);
generating a plurality of multi-condition alarms for the plurality of patients (514), each multi-condition alarm configured to output a respective audio signal when two or more physiological parameters of a respective patient exceed predefined thresholds concurrently;
in response to an input from a user of the multi-condition alarm system, configuring a first portion of the plurality of multi-condition alarms to be silenced when two or more physiological parameters of a respective patient exceed predefined thresholds concurrently (708), and configuring a second portion of the plurality of multi-condition alarms not to be silenced when two or more physiological parameters of a respective patient exceed predefined thresholds concurrently;
in a first condition where two or more physiological parameters of a first multi-condition alarm of the first portion of multi-condition alarms exceed respective predefined thresholds concurrently (804), not output the respective audio signal (808); and
in a second condition where two or more physiological parameters of a second multi-condition alarm of the second portion of multi-condition alarms exceed respective predefined thresholds concurrently, output the respective audio signal (812).

15. The method of claim 14, further comprising defining one or more respective breakthrough thresholds for one or more physiological parameters of a multi-condition alarm of the first portion of multi-condition alarms, the one or more respective breakthrough thresholds configured to generate a second audio signal when a physiological parameter of the one or more physiological parameters exceeds a respective breakthrough threshold.
